# EUROPÄISCHE PATENTSCHRIFT

(11) **EP 1 404 292 B1**
(45) Veröffentlichungstag und Bekanntmachung des Hinweises auf die Patenterteilung: **16.05.2007**
(21) Anmeldenummer: 02790564.5
(22) Anmeldetag: 05.07.2002
(51) Int. Cl.: A61K 8/36, A61K 8/37, A61K 8/39, A61Q 5/00, A61Q 5/12

(54) **ZUSAMMENSETZUNG AUF ÖLBASIS MIT EINEM GEHALT AN STERINEN**
OIL-BASED COMPOSITION COMPRISING STEROLS
COMPOSITION A BASE D'HUILE CONTENANT DES STEROLS

(30) Priorität: 10.07.2001 JP 2001209238
(43) Veröffentlichungstag der Anmeldung: 07.04.2004
(73) Patentinhaber: Cognis IP Management GmbH, 40589 Düsseldorf (DE)
(72) Erfinder: HATAZAKI, Shoichiro, Ibaraki 306-0204 (JP)
(74) Vertreter: Fabry, Bernd
(86) Internationale Anmeldenummer: PCT/IB2002/005037
(87) Internationale Veröffentlichungsnummer: WO 2003/035025

(56) Entgegenhaltungen:
- EP-A- 0 277 641
- WO-A-00/44344
- WO-A-93/02660
- US-A- 4 604 281
- KORHONEN M. ET AL.: "Rheological Properties of Creams with Four Different Surfactant Combinations- Effect of Storage Time and Conditions" INTERNATIONAL JOURNAL OF PHARMACEUTICS, Bd. 221, Nr. 1-2, 19. Juni 2001 (2001-06-19), Seiten 187-196, XP001146583
- TYLE P., FRANK S.G.: "Phytosterol Stabilized Emulsion: Interfacial Complexation and Structural Investigations" DRUG DEVELOPMENT AND INDUSTRIAL PHARMACY, Bd. 16, Nr. 10, 1990, Seiten 1605-1618, XP009008539
- TYLE P., FRANK S.G.: "Phytosterol Stabilized Emulsions: Correlation between Rheologic and Calorimetric Studies" DRUG DEVELOPMENT AND INDUSTRIAL PHARMACY, Bd. 17, Nr. 8, 1991, Seiten 1131-1141, XP009008540

## Beschreibung

### Gebiet

Die vorliegende Erfindung betrifft eine Zusammensetzung auf Ölbasis mit einem Gehalt an Sterinen.

### Stand der Technik

Bei Sterinen handelt es sich um allgemein bekannte, im Naturreich häufig vorkommenden Stoffe. Sie weisen im allgemeinen einen hohen Schmelzpunkt auf und sind in Wasser unlöslich. Auch in üblichen Fetten und Ölen sind sie nur bedingt löslich. Auf dem Gebiet der Kosmetik ist der Einsatz von Sterinen bekannt. Aufgrund diverser bei Tieren auftretender Seuchen und Krankheiten, wie beispielsweise Rinderwahnsinn (BSE) und Maul- und Klauenseuche, werden darüber hinaus in jüngster Zeit Rohstoffe tierischer Herkunft durch Stoffe pflanzlicher Herkunft zu ersetzt. So ist die Substitution von Wollwachs (Lanolin) durch pflanzliche Sterine bekannt.

Zum Einsatz von pflanzlichen Sterinen in Speiseölen und -fetten existiert eine reichhaltige Patentliteratur. Aus diesen Schriften sind jedoch bislang nur Zusammensetzungen in Kombination mit Glyceriden, deren Acylgruppen eine vergleichsweise hohe Kohlenstoffzahl aufweisen, bekannt. In kosmetischen Formulierungen führen diese jedoch häufig zu einem sehr fettigen und klebrigen Hautgefühl.

### Aufgabe der Erfindung

Aufgabe der vorliegenden Erfindung war es, Zusammensetzungen auf Ölbasis bereitzustellen, in der sich pflanzliche Sterine signifikant besser lösen und stabil gelöst bleiben. Die Zusammensetzungen sollen auch bei niedrigen Temperaturen eine hervorragende Fließfähigkeit aufweisen und bei der Verwendung als kosmetisches Mittel bzw. bei Einarbeitung in kosmetische Emulsionen ein sensorisch angenehmes Hautgefühl vermitteln.

Es wurde nun gefunden, dass die Löslichkeit von Sterinen durch Verwendung von Glycerinfettsäureesterkomponenten, in welcher der Anteil gesättigter Acylgruppen mit einer Kohlenstoffzahl von 12 und weniger bei mindestens 40% liegt und deren Hydroxylzahl (OH-Zahl) sich in einem definierten Bereich bewegt, signifikant verbessert wird.

Gegenstand der vorliegenden Erfindung sind daher Zusammensetzungen auf Ölbasis enthaltend
(a) Sterine und/oder Sterinester
(b) Glycerinfettsäureester
dadurch gekennzeichnet, daß die Glycerinfettsäureester wenigstens 40 % gesättigter Acylgruppen mit 12 oder weniger Kohlenstoffatomen enthalten und eine Hydroxylzahl zwischen 20 und 120 aufweisen.

Die erfindungsgemäßen Zusammensetzungen auf Ölbasis sind nahezu wasserfrei, d.h. daß sie lediglich rohstoffbedingt einen geringen Wasseranteil enthalten können, daß aber kein zusätzliches Wasser zugegeben wird. Der Wassergehalt beträgt weniger als 10 Gew.-% Wasser, vorzugsweise weniger als 6 Gew.-%, und insbesondere weniger als 3 Gew.-%.

Der Anteil der gesättigten Acylgruppen mit einer Kohlenstoffzahl von 12 und weniger in den Glycerinfettsäureestern liegt bei mindestens 40%, vorzugsweise jedoch bei mindestens 45%. Bei einem geringeren Anteil gesättigter Acylgruppen mit einer Kohlenstoffzahl von 12 und weniger in den Glycerinfettsäureestern neigen die Zusammensetzungen bei Zimmertemperatur verstärkt zur Verfestigung und zeigen eine geringere Löslichkeit für die Sterine. Die Glycerinfettsäureester weisen eine Hydroxylzahl (OH-Zahl) zwischen 20 und 120 auf. Bei einer Hydroxylzahl unter 20 ist ein Rückgang der Löslichkeit der Sterine zu beobachten. Eine Hydroxylzahl über 120 entspricht einem erhöhten Monoglyceridanteil im Glycerinfettsäureester und führt leichter zu Hautirritationen. Vorzugsweise weisen die Glycerinfettsäureester eine Hydroxylzahl im Bereich von 30 bis 100 auf.

Ein weiterer Gegenstand der vorliegenden Anmeldung ist die Verwendung von Glycerinfettsäureestern, die wenigstens 40 % gesättigter Acylgruppen mit 12 oder weniger Kohlenstoffatomen enthalten und eine OH-Zahl zwischen 20 und 120, insbesondere 30 bis 100, aufweisen, zur Lösung von Sterinen, insbesondere zur Verbesserung der Löslichkeit von Sterinen. Gegenstand der Anmeldung ist weiterhin die Verwendung der erfindungsgemäßen sterinhaltigen Zusammensetzungen auf Ölbasis zur kosmetischen Pflege der Haut.

Erfindungsgemäß besonders bevorzugte Zusammensetzungen sind dadurch gekennzeichnet, daß der Glycerinfettsäureester eine Monoglycerid-Anteil von höchstens 5 % aufweist. Diese Zusammensetzungen zeichnen sich durch ein vermindertes Hautirritationspotential aus. Erfindungsgemäß besonders bevorzugt ist ein Glycerincaprinsäureester, der unter der Bezeichnung Myritol^{®} 880 im Handel ist. Dieser zeigt neben guten Lösungseigenschaften für Sterine in kosmetischen Zusammensetzungen hinsichtlich der Sensorik deutlich verbesserte Eigenschaften.

### Sterine / Sterinester

Die Sterine (oft auch Sterole genannt) können in tierische und pflanzliche Vertreter unterteilt werden. Die im Tierreich vorkommenden Sterine nennt man Zoosterine. Wichtigste Beispiele sind Cholesterin, Lanosterin, sowie Zoosterine in der Seidenraupe, in Schwämmen (Spongosterin), Seesternen (Stellasterin), Seeigeln, Austern usw. Die pflanzlichen Sterine bezeichnet man als Phytosterine; ihre wichtigsten Vertreter sind Ergosterin, Stigmasterin u. Sitosterin. Gelegentlich trennt man von der Gruppe der Phytosterine die Sterine aus Pilzen u. Hefen als Mykosterine (z. B. Ergosterin, Fungisterin u. Zymosterin) ab. Erfindungsgemäß einsetzbar sind pflanzliche und tierische Sterine und Sterinester, insbesondere Sterinester mit C₆-C₂₄-Fettsäuren.

Erfindungsgemäß bevorzugt sind Zusammensetzungen, die Sterine oder Sterinester pflanzlicher Herkunft enthalten. Als Beispiel stehen Sitosterol, Campesterol, Brassicasterol, Lupenol, Stigmasterol, α-Spinasterol und Avennasterol, besonders bevorzugt sind β-Sitosterol und Campesterol. Zusammensetzungen, die 0,01 - 10 Gew.-% Sterine oder Sterinester, insbesondere pflanzlicher Herkunft enthalten, sind erfindungsgemäß bevorzugt. Insbesondere bevorzugt sind Mengen von 0,1 - 8 Gew.% und ganz besonders 1 - 7 Gew. %.

Soweit die Vorteile der Erfindung nicht beeinträchtigt werden, können den erfindungsgemäßen sterinhaltigen Zusammensetzungen auf Ölbasis andere üblicherweise in kosmetischen Mitteln eingesetzte Inhaltsstoffe zugesetzt werden. Als solche Inhaltsstoffe seien beispielhaft Antioxidationsmittel wie Butylhydroxytoluol und Tocopherol, UV-Absorber, Paraffine, Esteröle und weitere Öle tierischer und pflanzlicher Herkunft genannt. Die Zubereitung der erfindungsgemäßen Zusammensetzungen kann, falls nötig, durch Erwärmen und Rühren erfolgen. Vorzugsweise enthalten die erfindungsgemäßen Zubereitungen keine weiteren Ölkörper auf Glyceridbasis.

### Kosmetische und/ oder pharmazeutische Mittel

Die erfindungsgemäßen sterinhaltigen Zusammensetzungen auf Ölbasis, die eine besonders gute Löslichkeit für Sterine zeigen, können als Grundlage in kosmetische und/oder pharmazeutische Mittel eingearbeitet werden. Ein weiterer Gegenstand der vorliegend Anmeldung ist daher die Verwendung der Zusammensetzung zur Herstellung von kosmetischen und/oder pharmazeutischen O/W und W/O-Emulsionen. Gegenstand sind auch kosmetische und/oder pharmazeutische W/O und O/W-Emulsionen, die dadurch gekennzeichnet sind, daß sie 0,1 - 50 Gew.-% der sterinhaltigen Zusammensetzung auf Ölbasis enthalten. Vorzugsweise enthalten die Emulsionen 1 - 30 Gew.-% insbesondere 5 - 15 Gew.-% der sterinhaltigen Zusammensetzung auf Ölbasis.

Vorzugsweise handelt es sich hierbei um kosmetische Formulierungen zur Körperpflege, z. B. Cremes, Milchen, Lotionen, sprühbare Emulsionen, Produkte zur Eliminierung des Körpergeruchs etc. Die erfindungsgemäßen sterinhaltigen Zusammensetzungen auf Ölbasis lassen sich auch in tensidhaltige Formulierungen, wie z. B. Schaum- und Duschbädern, Haarshampoos und Pflegespülungen einsetzen. Je nach Applikationszweck enthalten die kosmetischen Formulierungen eine Reihe weiterer Hilfs- und Zusatzstoffe, wie beispielsweise Tenside, weitere Ölkörper, Emulgatoren, Periglanzwachse, Konsistenzgeber, Feuchthaltemittel, Verdickungsmittel, Überfettungsmittel, Stabilisatoren, Polymere, Siliconverbindungen, Fette, Wachse, Lecithine, Phospholipide, biogene Wirkstoffe, UV-Lichtschutzfaktoren, Antioxidantien, Deodorantien, Antitranspirantien, Antischuppenmittel, Filmbildner, Quellmittel, Insektenrepellentien, Selbstbräuner, Tyrosinaseinhibitoren (Depigmentierungsmittel), Hydrotrope, Solubilisatoren, Konservierungsmittel, Parfümöle, Farbstoffe, die nachstehend exemplarisch aufgelistet sind.

### Ölkörper

Körperpflegemittel, wie Cremes, Lotionen und Milchen, enthalten üblicherweise eine Reihe weiterer Ölkörper und Emollients, die dazu beitragen, die sensorischen Eigenschaften weiter zu optimieren. Die Ölkörper sind üblicherweise in einer Gesamtmenge von 1 - 40 Gew.-%, vorzugsweise 5 - 25 Gew.-% und insbesondere 5 - 15 Gew.-% enthalten. Als Ölkörper kommen beispielsweise Guerbetalkohole auf Basis von Fettalkoholen mit 6 bis 18, vorzugsweise 8 bis 10 Kohlenstoffatomen, Ester von linearen C₆-C₂₂-Fettsäuren mit linearen oder verzweigten C₆-C₂₂-Fettalkoholen bzw. Ester von verzweigten C₆-C₁₃-Carbonsäuren mit linearen oder verzweigten C₆-C₂₂-Fettalkoholen, wie z. B. Myristylmyristat, Myristylpalmitat, Myristylstearat, Myristylisostearat, Myristyloleat, Myristylbehenat, Myristylerucat, Cetylmyristat, Cetylpalmitat, Cetylstearat, Cetylisostearat, Cetyloleat, Cetylbehenat, Cetylerucat, Stearylmyristat, Stearylpalmitat, Stearylstearat, Stearylisostearat, Stearyloleat, Stearylbehenat, Stearylerucat, Isostearylmyristat, Isostearylpalmitat, Isostearylstearat, Isostearylisostearat, Isostearyloleat, Isostearylbehenat, Isostearyloleat, Oleylmyristat, Oleylpalmitat, Oleylstearat, Oleylisostearat, Oleyloleat, Oleylbehenat, Oleylerucat, Behenylmyristat, Behenylpalmitat, Behenylstearat, Behenylisostearat, Behenyloleat, Behenylbehenat, Behenylerucat, Erucylmyristat, Erucylpalmitat, Erucylstearat, Erucylisostearat, Erucyloleat, Erucylbehenat und Erucylerucat. Daneben eignen sich Ester von linearen C₆-C₂₂-Fettsäuren mit verzweigten Alkoholen, insbesondere 2-Ethylhexanol, Ester von C₁₈-C₃₈-Alkylhydroxycarbonsäuren mit linearen oder verzweigten C₆-C₂₂-Fettalkoholen, insbesondere Dioctyl Malate, Ester von linearen und/oder verzweigten Fettsäuren mit mehrwertigen Alkoholen (wie z. B. Propylenglycol, Dimerdiol oder Trimertriol) und/oder Guerbetalkoholen, Triglyceride auf Basis C₆-C₁₀-Fettsäuren, flüssige Mono-/Di-/Triglyceridmischungen auf Basis von C₆-C₁₈-Fettsäuren, Ester von C₆-C₂₂-Fettalkoholen und/oder Guerbetalkoholen mit aromatischen Carbonsäuren, insbesondere Benzoesäure, Ester von C₂-C₁₂-Dicarbonsäuren mit linearen oder verzweigten Alkoholen mit 1 bis 22 Kohlenstoffatomen oder Polyolen mit 2 bis 10 Kohlenstoffatomen und 2 bis 6 Hydroxylgruppen, pflanzliche Öle, verzweigte primäre Alkohole, substituierte Cyclohexane, lineare und verzweigte C₆-C₂₂-Fettalkoholcarbonate, wie z. B. Dicaprylylcarbonate (Cetiol® CC), Guerbetcarbonate auf Basis von Fettalkoholen mit 6 bis 18, vorzugsweise 8 bis 10 C Atomen, Ester der Benzoesäure mit linearen und/oder verzweigten C₆-C₂₂-Alkoholen (z. B. Finsolv® TN), lineare oder verzweigte, symmetrische oder unsymmetrische Dialkylether mit 6 bis 22 Kohlenstoffatomen pro Alkylgruppe, wie z. B. Dicaprylylether (Cetiol® OE), Ringöffnungsprodukte von epoxidierten Fettsäureestem mit Polyolen, Siliconöle (Cyclomethicone, Siliciummethicontypen u.a.) und/oder aliphatische bzw. naphthenische Kohlenwasserstoffe, wie z. B. wie Squalan, Squalen oder Dialkylcyclohexane in Betracht.

### Emulgatoren/Tenside

Als oberflächenaktive Stoffe können anionische, nichtionische, kationische und/oder amphotere bzw. zwitterionische Tenside/Emulgatoren enthalten sein. In tensidhaltigen kosmetischen Zubereitungen, wie beispielsweise Duschgelen, Schaumbädern, Shampoos etc. ist vorzugsweise wenigstens ein anionisches Tensid enthalten. In Körperpflegeprodukten, wie Cremes, Lotionen, etc. werden dagegen nichtionische Tenside bevorzugt. Der Anteil der Tenside liegt in Produkten zur Körperreinigung üblicherweise bei etwa 1 bis 30, vorzugsweise 5 bis 25 und insbesondere 10 bis 20 Gew.-% bezogen auf die Gesmatzusammensetzung. Körperpflegeprodukte enthalten vorzugsweise weniger als 15 Gew.-% und insbesondere weniger als 10 Gew.-% Tenside/Emulgatoren.

Typische Beispiele für anionische Tenside sind Seifen, Alkylbenzolsulfonate, Alkansulfonate, Olefinsulfonate, Alkylethersulfonate, Glycerinethersulfonate, α-Methylestersulfonate, Sulfofettsäuren, Alkylsulfate, Fettalkoholethersulfate, Glycerinethersulfate, Fettsäureethersulfate, Hydroxymischethersulfate, Monoglycerid(ether)sulfate, Fettsäureamid(ether)sulfate, Mono- und Dialkylsulfosuccinate, Mono- und Dialkylsulfosuccinamate, Sulfotriglyceride, Amidseifen, Ethercarbonsäuren und deren Salze, Fettsäureisethionate, Fettsäuresarcosinate. Fettsäuretauride, N-Acylaminosäuren, wie beispielsweise Acyllactylate, Acyltartrate, Acylglutamate und Acylaspartate, Alkyloligoglucosidsulfate, Proteinfettsäurekondensate (insbesondere pflanzliche Produkte auf Weizenbasis) und Alkyl(ether)phosphate. Sofern die anionischen Tenside Polyglycoletherketten enthalten, können diese eine konventionelle, vorzugsweise jedoch eine eingeengte Homologenverteilung aufweisen.

Als zwitterionische Tenside werden solche oberflächenaktiven Verbindungen bezeichnet, die im Molekül mindestens eine quartäre Ammoniumgruppe und mindestens eine Carboxylat- und eine Sulfonatgruppe tragen. Besonders geeignete zwitterionische Tenside sind die sogenannten Betaine wie die N-Alkyl-N,N-dimethylammoniumglycinate, beispielsweise das Kokosalkyldimethylammoniumglycinat, N-Acylaminopropyl-N,N-dimethylammoniumglycinate, beispielsweise das Kokosacylaminopropyldimethylammoniumglycinat, und 2-Alkyl-3-carboxylmethyl-3-hydroxyethylimidazoline mit jeweils 8 bis 18 C-Atomen in der Alkyl- oder Acylgruppe sowie das Kokosacylaminoethylhydroxyethylcarboxymethylglycinat. Besonders bevorzugt ist das unter der CTFA-Bezeichnung *Cocamidopropyl Betaine* bekannte Fettsäureamid-Derivat. Ebenfalls geeignete Emulgatoren sind **ampholytische** Tenside. Unter ampholytischen Tensiden werden solche oberflächenaktiven Verbindungen verstanden, die außer einer C_{8/18}-Alkyl- oder Acylgruppe im Molekül mindestens eine freie Aminogruppe und mindestens eine -COOH- oder -SO₃H-Gruppe enthalten und zur Ausbildung innerer Salze befähigt sind. Beispiele für geeignete ampholytische Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 18 C-Atomen in der Alkylgruppe. Besonders bevorzugte ampholytische Tenside sind das N-Kokosalkylaminopropionat, das Kokosacylaminoethylaminopro-pionat und das C_{12/18}-Acylsarcosin. Schließlich kommen auch Kationtenside als Emulgatoren in Betracht, wobei solche vom Typ der Esterquats, vorzugsweise methylquatemierte Difettsäuretriethanolaminester-Salze, besonders bevorzugt sind.

Besonders geeignet sind milde, d. h. besonders hautverträgliche Tenside, wie Fettalkoholpolyglycolethersulfate, Monoglyceridsulfate, Mono- und/oder Dialkylsulfosuccinate, Fettsäureisethionate, Fettsäuresarcosinate, Fettsäuretauride, Fettsäureglutamate, α-Olefinsulfonate, Ethercarbonsäuren, Alkyloligoglucoside, Fettsäureglucamide, Alkylamidobetaine, Amphoacetale und/oder Proteinfettsäurekondensate, letztere vorzugsweise auf Basis von Weizenproteinen.

Typische Beispiele für nichtionische Tenside sind Fettalkoholpolyglycolether, Alkylphenolpolyglycolether, Fettsäurepolyglycolester, Fettsäureamidpolyglycolether, Fettaminpolyglycolether, alkoxylierte Triglyceride, Mischether bzw. Mischformale, gegebenenfalls partiell oxidierte Alk(en)yloligoglykoside bzw. Glucoronsäurederivate, Fettsäure-N-alkylglucamide, Proteinhydrolysate (insbesondere pflanzliche Produkte auf Weizenbasis), Polyolfettsäureester, Zuckerester, Sorbitanester, Polysorbate und Aminoxide. Sofern die nichtionischen Tenside Polyglycoletherketten enthalten, können diese eine konventionelle, vorzugsweise jedoch eine eingeengte Homologenverteilung aufweisen. Unter den nichtionischen Tensiden sind u.a. Alkyl(en)oligoglucoside und Polyglycerinfettsäureester erfindungsgemäß bevorzugt. Hierzu zählen Produkte, die beispielsweise unter den Bezeichnungen Plantaren®, Plantacare®, Emulgade® PL 68/50, Dehymuls® PGPH und Eumulgin® VL 75 vermarktet werden.

### Fette und Wachse

Fette und Wachse werden den Körperpflegeprodukten als Pflegestoffe zugesetzt und auch, um die Konsistenz der Kosmetika zu erhöhen. Typische Beispiele für Fette sind Glyceride, d. h. feste oder flüssige pflanzliche oder tierische Produkte, die im wesentlichen aus gemischten Glycerinestern höherer Fettsäuren bestehen. Auch Fettsäurepartialglyceride, d. h. technische Mono- und/oder Diester des Glycerins mit Fettsäuren mit 12 bis 18 Kohlenstoffatomen wie etwa Glycerinmono/dilaurat, -palmitat oder -stearat kommen hierfür in Frage. Als Wachse kommen u. a. natürliche Wachse, wie z. B. Candelillawachs, Carnaubawachs, Japanwachs, Espartograswachs, Korkwachs, Guarumawachs, Reiskeimölwachs, Zuckerrohrwachs, Ouricurywachs, Montanwachs, Bienenwachs, Schellackwachs, Walrat, Lanolin (Wollwachs), Bürzelfett, Ceresin, Ozokerit (Erdwachs), Petrolatum, Paraffinwachse, Mikrowachse; chemisch modifizierte Wachse (Hartwachse), wie z. B. Montanesterwachse, Sasolwachse, hydrierte Jojobawachse sowie synthetische Wachse, wie z. B. Polyalkylenwachse und Polyethylenglycolwachse in Frage. Neben den Fetten kommen als Zusatzstoffe auch fettähnliche Substanzen, wie Lecithine und Phospholipide in Frage. Unter der Bezeichnung Lecithine versteht der Fachmann diejenigen Glycero-Phospholipide, die sich aus Fettsäuren, Glycerin, Phosphorsäure und Cholin durch Veresterung bilden. Lecithine werden in der Fachwelt daher auch häufig als Phosphatidylcholine (PC) bezeichnet. Als Beispiele für natürliche Lecithine seien die Kephaline genannt, die auch als Phosphatidsäuren bezeichnet werden und Derivate der 1,2-Diacyl-sn-glycerin-3-phosphorsäuren darstellen. Dem gegenüber versteht man unter Phospholipiden gewöhnlich Mono- und vorzugsweise Diester der Phosphorsäure mit Glycerin (Glycerinphosphate), die allgemein zu den Fetten gerechnet werden. Daneben kommen auch Sphingosine bzw. Sphingolipide in Frage.

### Perlglanzwachse

Als Perlglanzwachse kommen beispielsweise in Frage: Alkylenglycolester, speziell Ethylenglycoldistearat; Fettsäurealkanolamide, speziell Kokosfettsäurediethanolamid; Partialglyceride, speziell Stearinsäuremonoglycerid; Ester von mehrwertigen, gegebenenfalls hydroxy-substituierte Carbonsäuren mit Fettalkoholen mit 6 bis 22 Kohlenstoffatomen, speziell langkettige Ester der Weinsäure; Fettstoffe, wie beispielsweise Fettalkohole, Fettketone, Fettaldehyde, Fettether und Fettcarbonate, die in Summe mindestens 24 Kohlenstoffatome aufweisen, speziell Lauron und Distearylether; Fettsäuren wie Stearinsäure, Hydroxystearinsäure oder Behensäure, Ringöffnungsprodukte von Olefinepoxiden mit 12 bis 22 Kohlenstoffatomen mit Fettalkoholen mit 12 bis 22 Kohlenstoffatomen und/oder Polyolen mit 2 bis 15 Kohlenstoffatomen und 2 bis 10 Hydroxylgruppen sowie deren Mischungen.

### Konsistenzgeber und Verdickungsmittel

Als weitere Konsistenzgeber kommen in erster Linie Fettalkohole oder Hydroxyfettalkohole mit 12 bis 22 und vorzugsweise 16 bis 18 Kohlenstoffatomen und daneben Partialglyceride, Fettsäuren oder Hydroxyfettsäuren in Betracht. Bevorzugt ist eine Kombination dieser Stoffe mit Alkyloligoglucosiden und/oder Fettsäure-N-methylglucamiden gleicher Kettenlänge und/oder Polyglycerinpoly-12-hydroxystearaten. Geeignete Verdickungsmittel sind beispielsweise Aerosil-Typen (hydrophile Kieselsäuren), Polysaccharide, insbesondere Xanthan-Gum, Guar-Guar, Agar-Agar, Alginate und Tylosen, Carboxymethylcellulose und Hydroxyethyl- und Hydroxypropylcellulose, ferner höhermolekulare Polyethylenglycolmono- und -diester von Fettsäuren, Polyacrylate, (z. B. Carbopole® und Pemulen-Typen von Goodrich; Synthalene® von Sigma; Keltrol-Typen von Kelco; Sepigel-Typen von Seppic; Salcare-Typen von Allied Colloids), Polyacrylamide, Polymere, Polyvinylalkohol und Polyvinylpyrrolidon.

Als besonders wirkungsvoll haben sich auch Bentonite, wie z. B. Bentone® Gel VS-5PC (Rheox) erwiesen, bei dem es sich um eine Mischung aus Cyclopentasiloxan, Disteardimonium Hectorit und Propylencarbonat handelt. Weiter in Frage kommen Tenside, wie beispielsweise ethoxylierte Fettsäureglyceride, Ester von Fettsäuren mit Polyolen wie beispielsweise Pentaerythrit oder Trimethylolpropan, Fettalkoholethoxylate mit eingeengter Homologenverteilung oder Alkyloligoglucoside sowie Elektrolyte wie Kochsalz und Ammoniumchlorid.

### Überfettungsmittel

Als Überfettungsmittel können Substanzen wie beispielsweise Lanolin und Lecithin sowie polyethoxylierte oder acylierte Lanolin- und Lecithinderivate, Polyolfettsäureester, Monoglyceride und Fettsäurealkanolamide verwendet werden, wobei die letzteren gleichzeitig als Schaumstabilisatoren dienen.

### Stabilisatoren

Als Stabilisatoren können Metallsalze von Fettsäuren, wie z. B. Magnesium-, Aluminium- und/oder Zinkstearat bzw. -ricinoleat eingesetzt werden.

### Polymere

Geeignete kationische Polymere sind beispielsweise kationische Cellulosederivate, wie z. B. eine quaternierte Hydroxyethylcellulose, die unter der Bezeichnung Polymer JR® 400 von Amerchol erhältlich ist, kationische Stärke, Copolymere von Diallylammoniumsalzen und Acrylamiden, quaternierte Vinylpyrrolidon/Vinylimidazol-Polymere, wie z. B. Luviquat® (BASF), Kondensationsprodukte von Polyglycolen und Aminen, quaternierte Kollagenpolypeptide, wie beispielsweise Lauryldimonium Hydroxypropyl Hydrolyzed Collagen (Lamequat®L/Grünau), quaternierte Weizenpolypeptide, Polyethylenimin, kationische Siliconpolymere, wie z. B. Amodimethicone, Copolymere der Adipinsäure und Dimethylaminohydroxypropyldiethylentriamin (Cartaretine®/Sandoz), Copolymere der Acrylsäure mit Dimethyl-diallylammoniumchlorid (Merquat® 550/Chemviron), Polyaminopolyamide sowie deren vernetzte wasserlöslichen Polymere, kationische Chitinderivate wie beispielsweise quatemiertes Chitosan, gegebenenfalls mikrokristallin verteilt, Kondensationsprodukte aus Dihalogenalkylen, wie z. B. Dibrombutan mit Bisdialkylaminen, wie z. B. Bis-Dimethylamino-1,3-propan, kationischer Guar-Gum, wie z. B. Jaguar® CBS, Jaguar® C-17, Jaguar® C-16 der Firma Celanese, quaternierte Ammoniumsalz-Polymere, wie z. B. Mirapol® A-15, Mirapol® AD-1, Mirapol® AZ-1 der Firma Miranol.

Als anionische, zwitterionische, amphotere und nichtionische Polymere kommen beispielsweise Vinylacetat/Crotonsäure-Copolymere, Vinylpyrrolidon/Vinylacrylat-Copolymere, Vinylacetat/Butylmaleat/ Isobomylacrylat-Copolymere, Methylvinylether/Maleinsäureanhydrid-Copolymere und deren Ester, unvernetzte und mit Polyolen vernetzte Polyacrylsäuren, Acrylamidopropyltrimethylammoniumchlorid/ Acrylat-Copolymere, Octylacrylamid/Methylmeth-acrylat/tert.Butylaminoethylmethacrylat/2-Hydroxypropyl-methacrylat-Copolymere, Polyvinylpyrrolidon, Vinylpyrrolidon/Vinylacetat-Copolymere, Vinylpyrrolidon/ Dimethylaminoethylmethacrylat/Vinylcaprolactam-Terpolymere sowie gegebenenfalls derivatisierte Celluloseether und Silicone in Frage.

### Siliconverbindungen

Geeignete Siliconverbindungen sind beispielsweise Dimethylpolysiloxane, Methylphenylpolysiloxane, cyclische Silicone sowie amino-, fettsäure-, alkohol-, polyether-, epoxy-, fluor-, glykosid- und/oder alkylmodifizierte Siliconverbindungen, die bei Raumtemperatur sowohl flüssig als auch harzförmig vorliegen können. Weiterhin geeignet sind Simethicone, bei denen es sich um Mischungen aus Dimethiconen mit einer durchschnittlichen Kettenlänge von 200 bis 300 Dimethylsiloxan-Einheiten und hydrierten Silicaten handelt.

### UV-Lichtschutzfilter und Antioxidantien

Unter UV-Lichtschutzfaktoren sind beispielsweise bei Raumtemperatur flüssig oder kristallin vorliegende organische Substanzen (Lichtschutzfilter) zu verstehen, die in der Lage sind, ultraviolette Strahlen zu absorbieren und die aufgenommene Energie in Form längerwelliger Strahlung, z. B. Wärme wieder abzugeben. UVB-Filter können öllöslich oder wasserlöslich sein. Als öllösliche Substanzen sind z. B. zu nennen:
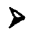 3-Benzylidencampher bzw. 3-Benzylidennorcampher und dessen Derivate, z. B. 3-(4-Methylbenzyliden)campher wie in der EP 0693471 B1 beschrieben
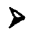 4-Aminobenzoesäurederivate, vorzugsweise 4-(Dimethylamino)benzoesäure-2-ethyl-hexylester, 4-(Dimethylamino)benzoesäure-2-octylester und 4-(Dimethylamino)benzoe-säureamylester
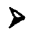 Ester der Zimtsäure, vorzugsweise 4-Methoxyzimtsäure-2-ethylhexylester, 4-Methoxyzimtsäure-propylester, 4-Methoxyzimtsäureisoamylester 2-Cyano-3,3-phenylzimtsäure-2-ethylhexylester (Octocrylene)
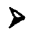 Ester der Salicylsäure, vorzugsweise Salicylsäure-2-ethylhexylester, Salicylsäure-4-iso-propylbenzylester, Salicylsäurehomomenthylester
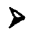 Derivate des Benzophenons, vorzugsweise 2-Hydroxy-4-methoxybenzophenon, 2-Hydroxy-4-methoxy-4'-methylbenzophenon, 2,2'-Dihydroxy-4-methoxybenzophenon
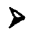 Ester der Benzalmalonsäure, vorzugsweise 4-Methoxybenzmalonsäuredi-2-ethylhexyl-ester
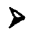 Triazinderivate, wie z. B. 2,4,6-Trianilino-(p-carbo-2'-ethyl-1'-hexyloxy)-1,3,5-triazin und Octyl-Triazon, wie in der **EP 0818450 A1** beschrieben oder Dioctyl Butamido Triazone (Uvasorb® HEB)
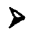 Propan-1,3-dione, wie z. B. 1-(4-tert.Butylphenyl)-3-(4'methoxyphenyl)propan-1,3-dion
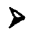 Ketotricyclo(5.2.1.0)decan-Derivate, wie in der **EP 0694521 B1** beschrieben.

Als wasserlösliche Substanzen kommen in Frage:
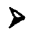 2-Phenylbenzimidazol-5-sulfonsäure und deren Alkali-, Erdalkali-, Ammonium-, Alkylammonium-, Alkanolammonium- und Glucammoniumsalze
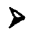 Sulfonsäurederivate von Benzophenonen, vorzugsweise 2-Hydroxy-4-methoxybenzo-phenon-5-sulfonsäure und ihre Salze
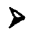 Suffonsäurederivate des 3-Benzylidencamphers, wie z.B. 4-(2-Oxo-3-bornylidenmethyl) benzolsulfonsäure und 2-Methyl-5-(2-oxo-3-bomyliden)sulfonsäure und deren Salze.

Als typische UV-A-Filter kommen insbesondere Derivate des Benzoylmethans in Frage, wie beispielsweise 1-(4'-tert.Butylphenyl)-3-(4'-methoxyphenyl)propan-1,3-dion, 4-tert.-Butyl-4'-methoxydibenzoylmethan (Parsol® 1789), 1-Phenyl-3-(4'-isopropylphenyl)-propan-1,3-dion sowie Enaminverbindungen, wie beschrieben in der **DE 19712033 A1** (BASF). Die UV-A und UV-B-Filter können selbstverständlich auch in Mischungen eingesetzt werden. Besonders günstige Kombinationen bestehen aus den Derivate des Benzoylmethans,, z. B. 4-tert.-Butyl-4'-methoxydibenzoylmethan (Parsol® 1789) und 2-Cyano-3,3-phenylzimtsäure-2-ethyl-hexylester (Octocrylene) in Kombination mit Ester der Zimtsäure, vorzugsweise 4-Methoxyzimtsäure-2-ethylhexylester und/oder 4-Methoxyzimtsäurepropylester und/oder 4-Methoxyzimtsäureisoamylester. Vorteilhaft werden derartige Kombinationen mit wasserlöslichen Filtern wie z. B. 2-Phenylbenzimidazol-5-sulfonsäure und deren Alkali-, Erdalkali-, Ammonium-, Alkylammonium-, Alkanolammonium- und Glucammoniumsalze kombiniert.

Neben den genannten löslichen Stoffen kommen für diesen Zweck auch unlösliche Lichtschutzpigmente, nämlich feindisperse Metalloxide bzw. Salze in Frage. Beispiele für geeignete Metalloxide sind insbesondere Zinkoxid und Titandioxid und daneben Oxide des Eisens, Zirkoniums, Siliciums, Mangans, Aluminiums und Cers sowie deren Gemische. Als Salze können Silicate (Talk), Bariumsulfat oder Zinkstearat eingesetzt werden. Die Oxide und Salze werden in Form der Pigmente für hautpflegende und hautschützende Emulsionen und dekorative Kosmetik verwendet. Die Partikel sollten dabei einen mittleren Durchmesser von weniger als 100 nm, vorzugsweise zwischen 5 und 50 nm und insbesondere zwischen 15 und 30 nm aufweisen. Sie können eine sphärische Form aufweisen, es können jedoch auch solche Partikel zum Einsatz kommen, die eine ellipsoide oder in sonstiger Weise von der sphärischen Gestalt abweichende Form besitzen. Die Pigmente können auch oberflächenbehandelt, d. h. hydrophilisiert oder hydrophobiert vorliegen. Typische Beispiele sind gecoatete Titandioxide, wie z. B. Titandioxid T 805 (Degussa) oder Eusolex® T2000 (Merck). Als hydrophobe Coatingmittel kommen dabei vor allem Silicone und dabei speziell Trialkoxyoctylsilane oder Simethicone in Frage. In Sonnenschutzmitteln werden bevorzugt sogenannte Mikro- oder Nanopigmente eingesetzt. Vorzugsweise wird mikronisiertes Zinkoxid verwendet.

Neben den beiden vorgenannten Gruppen primärer Lichtschutzstoffe können auch sekundäre Lichtschutzmittel vom Typ der Antioxidantien eingesetzt werden, die die photochemische Reaktionskette unterbrechen, welche ausgelöst wird, wenn UV-Strahlung in die Haut eindringt. Typische Beispiele hierfür sind Aminosäuren (z. B. Glycin, Histidin, Tyrosin, Tryptophan) und deren Derivate, Imidazole (z. B. Urocaninsäure) und deren Derivate, Peptide wie D,L-Camosin, D-Camosin, L-Camosin und deren Derivate (z. B. Anserin), Carotinoide, Carotine (z. B. α-Carotin, β-Carotin, Lycopin) und deren Derivate, Chlorogensäure und deren Derivate, Liponsäure und deren Derivate (z. B. Dihydroliponsäure), Aurothioglucose, Propylthiouracil und andere Thiole (z. B. Thioredoxin, Glutathion, Cystein, Cystin, Cystamin und deren Glycosyl-, N-Acetyl-, Methyl-, Ethyl-, Propyl-, Arnyl-, Butyl- und Lauryl-, Palmitoyl-, Oleyl-, γ-Linoleyl-, Cholesteryl- und Glycerylester) sowie deren Salze, Dilaurylthiodipropionat, Distearylthiodipropionat, Thiodipropionsäure und deren Derivate (Ester, Ether, Peptide, Lipide, Nukleotide, Nukleoside und Salze) sowie Sulfoximinverbindungen (z. B. Buthioninsulfoximine, Homocysteinsulfoximin, Butioninsulfone, Penta-, Hexa-, Heptathioninsulfoximin) in sehr geringen verträglichen Dosierungen (z. B. pmol bis µmol/kg), ferner (Metall)-Chelatoren (z. B. α-Hydroxyfettsäuren, Palmitinsäure, Phytinsäure, Lactoferrin), α-Hydroxysäuren (z. B. Citronensäure, Milchsäure, Äpfelsäure), Huminsäure, Gallensäure, Gallenextrakte, Bilirubin, Biliverdin, EDTA, EGTA und deren Derivate, ungesättigte Fettsäuren und deren Derivate (z. B. γ-Linolensäure, Linolsäure, Ölsäure), Folsäure und deren Derivate, Ubichinon und Ubichinol und deren Derivate, Vitamin C und Derivate (z. B. Ascorbylpalmitat, Mg-Ascorbylphosphat, Ascorbylacetat), Tocopherole und Derivate (z. B. Vitamin-E-acetat), Vitamin A und Derivate (Vitamin-A-palmitat) sowie Koniferylbenzoat des Benzoeharzes, Rutinsäure und deren Derivate, α-Glycosylrutin, Ferulasäure, Furfurylidenglucitol, Carnosin, Butylhydroxytoluol, Butylhydroxyanisol, Nordihydroguajakharzsäure, Nordihydroguajaretsäure, Trihydroxybutyrophenon, Harnsäure und deren Derivate, Mannose und deren Derivate, Superoxid-Dismutase, Zink und dessen Derivate (z. B. ZnO, ZnSO₄) Selen und dessen Derivate (z. B. Selen-Methionin), Stilbene und deren Derivate (z. B. Stilbenoxid, trans-Stilbenoxid) und die erfindungsgemäß geeigneten Derivate (Salze, Ester, Ether, Zucker, Nukleotide, Nukleoside, Peptide und Lipide) dieser genannten Wirkstoffe.

### Biogene Wirkstoffe

Unter biogenen Wirkstoffen sind beispielsweise Tocopherol, Tocopherolacetat, Tocopherolpalmitat, Ascorbinsäure, (Desoxy)Ribonucleinsäure und deren Fragmentierungsprodukte, β-Glucane, Retinol, Bisabolol, Allantoin, Phytantriol, Panthenol, AHA-Säuren, Aminosäuren, Ceramide, Pseudoceramide, essentielle Öle, Pflanzenextrakte, wie z. B. Prunusextrakt, Bambaranussextrakt und Vitaminkomplexe zu verstehen.

### Deodorantien und keimhemmende Mittel

Kosmetische Deodorantien (Desodorantien) wirken Körpergerüchen entgegen, überdecken oder beseitigen sie. Körpergerüche entstehen durch die Einwirkung von Hautbakterien auf apokrinen Schweiß, wobei unangenehm riechende Abbauprodukte gebildet werden. Dementsprechend enthalten Deodorantien Wirkstoffe, die als keimhemmende Mittel, Enzyminhibitoren, Geruchsabsorber oder Geruchsüberdecker fungieren.
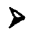 Keimhemmende Mittel
   Als keimhemmende Mittel sind grundsätzlich alle gegen grampositive Bakterien wirksamen Stoffe geeignet, wie z. B. 4-Hydroxybenzoesäure und ihre Salze und Ester, N-(4-Chlorphenyl)-N'-(3,4-dichlorphenyl)hamstoff, 2,4,4'-Trichlor-2'-hydroxy-diphenylether (Triclosan), 4-Chlor-3,5-dimethylphenol, 2,2'-Methylen-bis(6-brom-4-chlorphenol), 3-Methyl-4-(1-methylethyl)-phenol, 2-Benzyl-4-chlorphenol, 3-(4-Chlorphenoxy)-1,2-propandiol, 3-lod-2-propinylbutylcarbamat, Chlorhexidin, 3,4,4'-Trichlorcarbanilid (TTC), antibakterielle Riechstoffe, Thymol, Thymianöl, Eugenol, Nelkenöl,
   Menthol, Minzöl, Farnesol, Phenoxyethanol, Glycerinmonocaprinat, Glycerinmonocaprylat, Glycerinmonolaurat (GML), Diglycerinmonocaprinat (DMC), Salicylsäure-N-alkylamide wie z. B. Salicylsäure-n-octylamid oder Salicylsäure-n-decylamid.
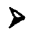 Enzyminhibitoren
   Als Enzyminhibitoren sind beispielsweise Esteraseinhibitoren geeignet. Hierbei handelt es sich vorzugsweise um Trialkylcitrate wie Trimethylcitrat, Tripropylcitrat, Triisopropylcitrat, Tributylcitrat und insbesondere Triethylcitrat (Hydagen® CAT). Die Stoffe inhibieren die Enzymaktivität und reduzieren dadurch die Geruchsbildung. Weitere Stoffe, die als Esteraseinhibitoren in Betracht kommen, sind Sterolsulfate oder -phosphate, wie beispielsweise Lanosterin-, Cholesterin-, Campesterin-, Stigmasterin- und Sitosterinsulfat bzw -phosphat, Dicarbonsäuren und deren Ester, wie beispielsweise Glutarsäure, Glutarsäuremonoethylester, Glutarsäurediethylester, Adipinsäure, Adipinsäuremonoethylester, Adipinsäurediethylester, Malonsäure und Malonsäurediethylester, Hydroxycarbonsäuren und deren Ester wie beispielsweise Citronensäure, Äpfelsäure, Weinsäure oder Weinsäurediethylester, sowie Zinkglycinat.
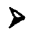 Geruchsabsorber
   Als Geruchsabsorber eignen sich Stoffe, die geruchsbildende Verbindungen aufnehmen und weitgehend festhalten können. Sie senken den Partialdruck der einzelnen Komponenten und verringern so auch ihre Ausbreitungsgeschwindigkeit. Wichtig ist, dass dabei Parfums unbeeinträchtigt bleiben müssen. Geruchsabsorber haben keine Wirksamkeit gegen Bakterien. Sie enthalten beispielsweise als Hauptbestandteil ein komplexes Zinksalz der Ricinolsäure oder spezielle, weitgehend geruchsneutrale Duftstoffe, die dem Fachmann als "Fixateure" bekannt sind, wie z. B. Extrakte von Labdanum bzw. Styrax oder bestimmte Abietinsäurederivate. Als Geruchsüberdecker fungieren Riechstoffe oder Parfümöle, die zusätzlich zu ihrer Funktion als Geruchsüberdecker den Deodorantien ihre jeweilige Duftnote verleihen. Als Parfümöle seien beispielsweise genannt Gemische aus natürlichen und synthetischen Riechstoffen. Natürliche Riechstoffe sind Extrakte von Blüten, Stengeln und Blättern, Früchten, Fruchtschalen, Wurzeln, Hölzern, Kräutern und Gräsem, Nadeln und Zweigen sowie Harzen und Balsamen. Weiterhin kommen tierische Rohstoffe in Frage, wie beispielsweise Zibet und Castoreum. Typische synthetische Riechstoffverbindungen sind Produkte vom Typ der Ester, Ether, Aldehyde, Ketone, Alkohole und Kohlenwasserstoffe. Riechstoffverbindungen vom Typ der Ester sind z. B. Benzylacetat, p-tert.-Butylcyclohexylacetat, Linalylacetat, Phenylethylacetat, Linalylbenzoat, Benzylformiat, Allylcyclohexylpropionat, Styrallylpropionat und Benzylsalicylat. Zu den Ethern zählen beispielsweise Benzylethylether, zu den Aldehyden z. B. die linearen Alkanale mit 8 bis 18 Kohlenstoffatomen, Citral, Citronellal, Citronellyloxyacetaldehyd, Cyclamenaldehyd, Hydroxycitronellal, Lilial und Bourgeonal, zu den Ketonen z. B. die Jonone und Methylcedrylketon, zu den Alkoholen Anethol, Citronellol, Eugenol, Isoeugenol, Geraniol, Linalool, Phenylethylalkohol und Terpineol, zu den Kohlenwasserstoffen gehören hauptsächlich die Terpene und Balsame. Bevorzugt werden jedoch Mischungen verschiedener Riechstoffe verwendet, die gemeinsam eine ansprechende Duftnote erzeugen. Auch ätherische Öle geringerer Flüchtigkeit, die meist als Aromakomponenten verwendet werden, eignen sich als Parfümöle, z. B. Salbeiöl, Kamillenöl, Nelkenöl, Melissenöl, Minzenöl, Zimtblätteröl, Lindenblütenöl, Wacholderbeerenöl, Vetiveröl, Olibanöl, Galbanumöl, Labdanumöl und Lavandinöl. Vorzugsweise werden Bergamotteöl, Dihydromyrcenol, Lilial, Lyral, Citronellol, Phenylethylalkohol, α-Hexylzimtaldehyd, Geraniol, Benzylaceton, Cyclamenaldehyd, Linalool, Boisambrene Forte, Ambroxan, Indol, Hedione, Sandelice, Citronenöl, Mandarinenöl, Orangenöl, Allylamylglycolat, Cyclovertal, Lavandinöl, Muskateller Salbeiöl, β-Damascone, Geraniumöl Bourbon, Cyclohexylsalicylat, Vertofix Coeur, Fixolide NP, Evemyl, Iraldein gamma, Phenylessigsäure, Geranylacetat, Benzylacetat, Rosenoxid, Romilat, Irotyl und Floramat allein oder in Mischungen, eingesetzt.
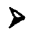 Antitranspirantien
   Antitranspirantien (Antiperspirantien) reduzieren durch Beeinflussung der Aktivität der ekkrinen Schweißdrüsen die Schweißbildung, und wirken somit Achselnässe und Körpergeruch entgegen. Wässrige oder wasserfreie Formulierungen von Antitranspirantien enthalten typischerweise folgende Inhaltsstoffe:
   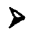 adstringierende Wirkstoffe
   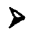 Ölkomponenten
   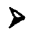 nichtionische Emulgatoren
   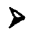 Coemulgatoren
   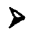 Konsistenzgeber
   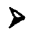 Hilfsstoffe wie z. B. Verdicker oder Komplexierungsmittel und/oder
   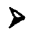 nichtwässrige Lösungsmittel wie z. B. Ethanol, Propylenglykol und/oder Glycerin.
   Als adstringierende Antitranspirant-Wirkstoffe eignen sich vor allem Salze des Aluminiums, Zirkoniums oder des Zinks. Solche geeigneten antihydrotisch wirksamen Wirkstoffe sind z. B. Aluminiumchlorid, Aluminiumchlorhydrat, Aluminiumdichlorhydrat, Aluminiumsesquichlorhydrat und deren Komplexverbindungen z. B. mit Propylenglycol-1,2. Aluminiumhydroxyallantoinat, Aluminiumchloridtartrat, Aluminium-Zirkonium-Trichlorohydrat, Aluminium-Zirkonium-tetrachlorohydrat, Aluminium-Zirkonium-pentachlorohydrat und deren Komplexverbindungen z. B. mit Aminosäuren wie Glycin. Daneben können in Antitranspirantien übliche öllösliche und wasserlösliche Hilfsmittel in geringeren Mengen enthalten sein.
   Solche öllöslichen Hilfsmittel können z. B. sein:
   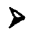 entzündungshemmende, hautschützende oder wohlriechende ätherische Öle
   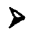 synthetische hautschützende Wirkstoffe und/oder
   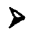 öllösliche Parfümöle.

Übliche wasserlösliche Zusätze sind z. B. Konservierungsmittel, wasserlösliche Duftstoffe, pH-Wert-Stellmittel, z. B. Puffergemische, wasserlösliche Verdickungsmittel, z. B. wasserlösliche natürliche oder synthetische Polymere wie z. B. Xanthan-Gum, Hydroxyethylcellulose, Polyvinylpyrrolidon oder hochmolekulare Polyethylenoxide.

### Filmbildner

Gebräuchliche Filmbildner sind beispielsweise Chitosan, mikrokristallines Chitosän, quaterniertes Chitosan, Polyvinylpyrrolidon, Vinylpyrrolidon-Vinylacetat-Copolymerisate, Polymere der Acrylsäurereihe, quaternäre Cellulose-Derivate, Kollagen, Hyaluronsäure bzw. deren Salze und ähnliche Verbindungen.

### Antischuppenwirkstoffe

Als Antischuppenwirkstoffe kommen Pirocton Olamin (1-Hydroxy-4-methyl-6-(2,4,4-trimythylpentyl)-2-(1H)-pyridinonmonoethanolaminsalz), Baypival® (Climbazole), Ketoconazol®, (4-Acetyl-1-{-4-[2-(2.4-dichlorphenyl) r-2-(1H-imidazol-1-ylmethyl)-1,3-dioxylan-c-4-ylmethoxyphenyl}piperazin, Ketoconazol, Elubiol, Selendisulfid, Schwefel kolloidal, Schwefelpolyehtylenglykolsorbitanmonooleat, Schwefelrizinolpolyehtoxylat, Schwefelteer Destillate, Salicylsäure (bzw. in Kombination mit Hexachlorophen), Undexylensäure-Monoethanolamid-Sulfosuccinat-Na-Salz, Lamepon® UD (Protein-Undecylensäurekondensat), Zinkpyrithion, Aluminiumpyrithion und Magnesiumpyrithion / Dipyrithion-Magnesiumsulfat in Frage.

### Quellmittel

Als Quellmittel für wässrige Phasen können Montmorillonite, Clay Mineralstoffe, Pemulen sowie alkylmodifizierte Carbopoltypen (Goodrich) dienen. Weitere geeignete Polymere bzw. Quellmittel können der Übersicht von R.Lochhead in **Cosm.Toil. 108, 95 (1993)** entnommen werden.

### Insekten-Repellentien

Als Insekten-Repellentien kommen beispielsweise N,N-Diethyl-m-toluamid, 1,2-Pentandiol oder 3-(N-n-Butyl-N-acetyl-amino)-propionic acid-ethyl ester), welches unter der Bezeichnung Insect Repellent® 3535 von der Merck KGaA vertrieben wird, sowie Butylacetylaminopropionate in Frage.

### Selbstbräuner und Depigmentierungsmittel

Als Selbstbräuner eignet sich Dihydroxyaceton. Als Tyrosinhinbitoren, die die Bildung von Melanin verhindern und Anwendung in Depigmentierungsmitteln finden, kommen beispielsweise Arbutin, Ferulasäure, Kojisäure, Cumarinsäure und Ascorbinsäure (Vitamin C) in Frage.

### Hydrotrope

Zur Verbesserung des Fließverhaltens können ferner Hydrotrope, wie beispielsweise Ethanol, Isopropylalkohol, oder Polyole eingesetzt werden. Polyole, die hier in Betracht kommen, besitzen vorzugsweise 2 bis 15 Kohlenstoffatome und mindestens zwei Hydroxylgruppen. Die Polyole können noch weitere funktionelle Gruppen, insbesondere Aminogruppen, enthalten bzw. mit Stickstoff modifiziert sein. Typische Beispiele sind:
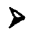 Glycerin
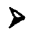 Alkylenglycole, wie beispielsweise Ethylenglycol, Diethylenglycol, Propylenglycol, Butylenglycol, Hexylenglycol sowie Polyethylenglycole mit einem durchschnittlichen Molekulargewicht von 100 bis 1.000 Dalton
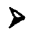 technische Oligoglyceringemische mit einem Eigenkondensationsgrad von 1,5 bis 10 wie etwa technische Diglyceringemische mit einem Diglyceringehalt von 40 bis 50 Gew.-%
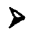 Methyolverbindungen, wie insbesondere Trimethylolethan, Trimethylolpropan, Trimethylolbutan, Pentaerythrit und Dipentaerythrit
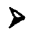 Niedrigalkylglucoside, insbesondere solche mit 1 bis 8 Kohlenstoffen im Alkylrest, wie beispielsweise Methyl- und Butylglucosid
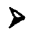 Zuckeralkohole mit 5 bis 12 Kohlenstoffatomen, wie beispielsweise Sorbit oder Mannit
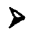 Zucker mit 5 bis 12 Kohlenstoffatomen, wie beispielsweise Glucose oder Saccharose
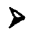 Aminozucker, wie beispielsweise Glucamin
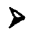 Dialkoholamine, wie Diethanolamin oder 2-Amino-1,3-propandiol.

### Konservierungsmittel

Als Konservierungsmittel eignen sich beispielsweise Phenoxyethanol, Formaldehydlösung, Parabene, Pentandiol oder Sorbinsäure sowie die unter der Bezeichnung Surfacine® bekannten Silberkomplexe und die in Anlage 6, Teil A und B der Kosmetikverordnung aufgeführten weiteren Stoffklassen.

### Parfümöle und Aromen

Als Parfümöle seien genannt Gemische aus natürlichen und synthetischen Riechstoffen. Natürliche Riechstoffe sind Extrakte von Blüten (Lilie, Lavendel, Rosen, Jasmin, Neroli, Ylang-Ylang), Stengeln und Blättern (Geranium, Patchouli, Petitgrain), Früchten (Anis, Koriander, Kümmel, Wacholder), Fruchtschalen (Bergamotte, Zitrone, Orangen), Wurzeln (Macis, Angelica, Sellerie, Kardamon, Costus, Iris, Calmus), Hölzern (Pinien-, Sandel-, Guajak-, Zedem-, Rosenholz), Kräutern und Gräsem (Estragon, Lemongras, Salbei, Thymian), Nadeln und Zweigen (Fichte, Tanne, Kiefer, Latschen), Harzen und Balsamen (Galbanum, Elemi, Benzoe, Myrrhe, Olibanum, Opoponax). Weiterhin kommen tierische Rohstoffe in Frage, wie beispielsweise Zibet und Castoreum. Typische synthetische Riechstoffverbindungen sind Produkte vom Typ der Ester, Ether, Aldehyde, Ketone, Alkohole und Kohlenwasserstoffe. Riechstoffverbindungen vom Typ der Ester sind z. B. Benzylacetat, Phenoxyethylisobutyrat, p-tert.-Butylcyclohexylacetat, Linalylacetat, Dimethylbenzylcarbinylacetat, Phenylethylacetat, Linalylbenzoat, Benzylformiat, Ethylmethylphenylglycinat, Allylcyclohexylpropionat, Styrallylpropionat und Benzylsalicylat. Zu den Ethern zählen beispielsweise Benzylethylether, zu den Aldehyden z. B. die linearen Alkanale mit 8 bis 18 Kohlenstoffatomen, Citral, Citronellal, Citronellyloxyacetaldehyd, Cyclamenaldehyd, Hydroxycitronellal, Lilial und Bourgeonal, zu den Ketonen z. B. die Jonone, α-Isomethylionon und Methylcedrylketon, zu den Alkoholen Anethol, Citronellol, Eugenol, Isoeugenol, Geraniol, Linalool, Phenylethylalkohol und Terpineol, zu den Kohlenwasserstoffen gehören hauptsächlich die Terpene und Balsame. Bevorzugt werden jedoch Mischungen verschiedener Riechstoffe verwendet, die gemeinsam eine ansprechende Duftnote erzeugen. Auch ätherische Öle geringerer Flüchtigkeit, die meist als Aromakomponenten verwendet werden, eignen sich als Parfümöle, z. B. Salbeiöl, Kamillenöl, Nelkenöl, Melissenöl, Minzenöl, Zimtblätteröl, Lindenblütenöl, Wacholderbeerenöl, Vetiveröl, Olibanöl, Galbanumöl, Labolanumöl und Lavandinöl. Vorzugsweise werden Bergamotteöl, Dihydromyrcenol, Lilial, Lyral, Citronellol, Phenylethylalkohol, α-Hexylzimtaldehyd, Geraniol, Benzylaceton, Cyclamenaldehyd, Linalool, Boisambrene Forte, Ambroxan, Indol, Hedione, Sandelice, Citronenöl, Mandarinenöl, Orangenöl, Allylamylglycolat, Cyclovertal, Lavandinöl, Muskateller Salbeiöl, β-Damascone, Geraniumöl Bourbon, Cyclohexylsalicylat, Vertofix Coeur, Iso-E-Super, Fixolide NP, Evemyl, Iraldein gamma, Phenylessigsäure, Geranylacetat, Benzylacetat, Rosenoxid, Romilllat, Irotyl und Floramat allein oder in Mischungen, eingesetzt.

Als Aromen kommen beispielsweise Pfefferminzöl, Krauseminzöl, Anisöl, Stemanisöl, Kümmelöl, Eukalyptusöl, Fenchelöl, Citronenöl, Wintergrünöl, Nelkenöl, Menthol und dergleichen in Frage.

### Farbstoffe

Als Farbstoffe können die für kosmetische Zwecke geeigneten und zugelassenen Substanzen verwendet werden. Beispiele sind Kochenillerot A (C.I. 16255), Patentblau V (C.I.42051), Indigotin (C.I.73015), Chlorophyllin (C.I.75810), Chinolingelb (C.I.47005), Titandioxid (C.I.77891), Indanthrenblau RS (C.I. 69800) und Krapplack (C.I.58000). Als Lumineszenzfarbstoff kann auch Luminol enthalten sein. Diese Farbstoffe werden üblicherweise in Konzentrationen von 0,001 bis 0,1 Gew.-%, bezogen auf die gesamte Mischung, eingesetzt.

### Beispiele

Im folgenden wird die Erfindung durch Ausführungsbeispiele illustriert, ohne hierdurch den Gegenstand der Erfindung zu beschränken.

### 1. Bewertung der Löslichkeit am Beispiel von Generol® R (Phytosterin) in verschiedenen Ölen

Es wurden jeweils 1 Gew.-%, 3 Gew.-%, 5 Gew.-% und 7 Gew.-% Generol® R in verschiedenen Glycerinfettsäurestern gelöst und nach 24 Stunden bezüglich der Löslichkeit optisch bewertet (Tabelle 1); ***gut*** = Zubereitung ist klar geblieben, keine Trübung; ***mittel*** = Zubereitung hat sich eingetrübt; ***schlecht*** = Zubereitung hat sich eingetrübt und verfestigt. Die Löslichkeitsreihe zeigt den Einfluß der Kettenlänge und der OH-Zahl auf die Löslichkeit.

**Tabelle 1**

| | **Löslichkeit** | | | |
|---|---|---|---|---|
| **Generol® R** | **1%** | **3%** | **5%** | **7%** |
| **Glycerintricaprinsäureester; OH-Zahl = 3; (Myritol® 888)** | mittel | schlecht | schlecht | schlecht |
| **Glycerintricaprinsäureester; OH-Zahl = 80; (Myritol® 880)** | gut | gut | gut | gut |
| **Glycerinmonocaprinsäureester (OH-Zahl = 500)** | gut | gut | gut | gut |
| **Glycerintriölsäureester; OH-Zahl = 80** | gut | gut | gut | gut |

### 2. Bewertung des Anwendungsgefühls erfindungsgemäßer Zusammensetzungen am Beispiel von Emulsionen

Es wurden kosmetische Emulsionen/Cremes (Tabelle 2) hergestellt und sensorisch durch ein Panel von 20 Experten hinsichtlich Feuchthaltegefühl, Klebrigkeit und Irritation beurteilt.

**Feuchthaltegefühl: *gut*** = mindestens 10 Experten beurteilten das Feuchthaltegefühl als gut; ***mittel*** = 5 bis 9 Experten beurteilten das Feuchthaltegefühl als gut; ***schlecht*** = weniger als 5 Experten beurteilten das Feuchthaltegefühl als gut.

**Klebrigkeit: *gut*** = weniger als 5 Experten beurteilten die Zubereitung als klebrig; ***mittel*** = 5 bis 9 Experten beurteilten die Zubereitung als klebrig; ***schlecht*** = mindestens 10 Experten beurteilten die Zubereitung als klebrig.

**Irritation: *gut*** = keiner der Experten empfand die Zubereitung als irritierend; **mittel** = 1 bis 4 Experten empfanden die Zubereitung als irritierend; ***schlecht*** = mindestens 5 empfanden die Zubereitung als irritierend.

### Tabelle 2: Kosmetische Emulsionen und sensorische Beurteilung

Die Mengenangaben in nachfolgenden Beispielen beziehen sich, soweit nicht anders angegeben, auf Gew.-% der handelsüblichen Substanzen in der Gesamtzusammensetzung. Beispiel 1 ist erfindungsgemäß, die Formulierungen V1, V2 und V3 stellen Vergleichsbeispiele dar.

**Tabelle 2**

| **Zusammensetzung/Beurteilung** | **1** | **V1** | **V2** | **V3** |
|---|---|---|---|---|
| **Eumulgin^{®} VL 75** | 3,5 | 3,5 | 3,5 | 3,5 |
| **Cetiol^{®} CC** | 7 | 7 | 7 | 7 |
| **Myritol^{®} 880; OH-Zahl = 80** | 7 | | | |
| **Myritol^{®} 888; OH-Zahl = 3** | | 7 | | |
| **Glycerinmonocaprinsäureester; OH-Zahl = 500** | | | 7 | |
| **Glycerintriölsäureester; OH-Zahl = 80** | | | | 7 |
| **Dipropylenglykol** | 5 | 5 | 5 | 5 |
| **Generol^{®} R** | 5 | 5 | 5 | 5 |
| **Carbopol^{®} 980** | 0,3 | 0,3 | 0,3 | 0,3 |
| **Konservierungsmittel** | 0,3 | 0,3 | 0,3 | 0,3 |
| **Kaliumhydroxid** | q.s. | q.s. | q.s. | q.s. |
| **Wasser** | ad 100 | ad 100 | ad 100 | ad 100 |

| ***Beurteilung*** | | | | |
|---|---|---|---|---|
| *Feuchtigkeit* | gut | mittel | gut | gut |
| *Klebrigkeit* | gut | mittel | gut | schlecht |
| *Irritation* | gut | gut | schlecht | gut |

Die Ergebnisse aus Tabelle 1 und 2 zeigen, dass eine gute Löslichkeit für Sterine in Kombination mit einem verbesserten sensorischen Eigenschaftsprofil durch bestimmte Glycerinfettsäureester erzielt werden kann. Diese Glycerinfettsäureester enthalten wenigstens 40 % gesättigter Acylgruppen mit 12 oder weniger Kohlenstoffatomen und weisen eine Hydroxylzahl zwischen 20 und 120 auf.

### Anhang

1) Carbopol® 980
   INCI: Carbomer
   Hersteller: Noveon
2) Cetiol® CC
   INCI: Dicaprylyl Carbonate
   Hersteller: Cognis Deutschland GmbH & Co. KG
3) Eumulgin® VL 75
   INCI: Lauryl Glucoside and Polyglyceryl-2 Dipolyhydroxystearate and Glycerin
   Hersteller: Cognis Deutschland GmbH & Co. KG
4) Generol® R
   INCI: Brassica campestris sterols
   Hersteller: Cognis Deutschland GmbH & Co. KG
5) Myritol® 880
   INCI: Tricaprylin
   Hersteller: Cognis Deutschland GmbH & Co. KG
6) Myritol® 888
   INCI: Tricaprylin
   Hersteller: Cognis Japan Ltd.

## Patentansprüche

1. Zusammensetzung auf Ölbasis enthaltend
(a) Sterine und/oder Sterinester
(b) Glycerinfettsäureester
**dadurch gekennzeichnet, daß** sie und weniger als 10 Gew.-% Wasser umfasst und die Glycerinfettsäureester wenigstens 40 % gesättigter Acylgruppen mit 12 oder weniger Kohlenstoffatomen enthalten und eine Hydroxylzahl zwischen 20 und 120 aufweisen.

2. Zusammensetzung gemäß Anspruch 1, **dadurch gekennzeichnet, daß** die Glycerinfettsäureester einen Monoglycerid-Anteil von höchstens 5 % aufweist.

3. Zusammensetzung gemäß einem der Ansprüche 1 bis 2, **dadurch gekennzeichnet, daß** sie Sterine oder Sterinester pflanzlicher Herkunft enthalten.

4. Zusammensetzung gemäß wenigstens einem der Ansprüche 1 bis 3, **dadurch gekennzeichnet, daß** sie 0,01-10 Gew.-% Sterine oder Sterinester enthalten.

5. Verwendung von Glycerinfettsäureestern, die wenigstens 40 % gesättigter Acylgruppen mit 12 oder weniger Kohlenstoffatomen enthalten und eine OH-Zahl zwischen 20 und 120, insbesondere 30 und 100, aufweisen, zur Lösung von Sterinen.

6. Verwendung der Zusammensetzung gemäß wenigstens einem der Ansprüche 1 bis 4 zur nicht therapeutischen, kasmetischen Pflege der Haut.

7. Verwendung der Zusammensetzung gemäß wenigstens einem der Ansprüche 1 bis 4 zur Herstellung von kosmetischen und/oder pharmazeutischen O/W- oder W/O-Emulsionen.

8. Kosmetische und/oder pharmazeutische O/W oder W/O-Emulsionen, **dadurch gekennzeichnet, daß** sie 0,1 - 50 Gew.-% der Zusammensetzung gemäß einem der Ansprüche 1 bis 4 enthalten.

## Claims

1. Oil-based composition containing
(a) sterols and/or sterol esters
(b) glycerol fatty acid esters,
**characterized in that** they contain less than 10% by weight water and the glycerol fatty acid esters contain at least 40% of saturated acyl groups containing 12 or fewer carbon atoms and have a hydroxyl value of 20 to 120.

2. A composition as claimed in claim 1, **characterized in that** the glycerol fatty acid ester has a percentage monoglyceride content of at most 5%.

3. A composition as claimed in claim 1 or 2, **characterized in that** it contains sterols or sterol esters of vegetable origin.

4. A composition as claimed in at least one of claims 1 to 3, **characterized in that** it contains 0.01 to 10% by weight sterols or sterol esters.

5. The use of glycerol fatty acid esters which contain at least 40% of saturated acyl groups containing 12 or fewer carbon atoms and have an OH value of 20 to 120, more particularly 30 to 100, for dissolving sterols.

6. The use of the composition claimed in at least one of claims 1 to 4 for non-therapeutic, cosmetic skin care.

7. The use of the composition claimed in at least one of claims 1 to 4 for the production of cosmetic and/or pharmaceutical o/w or w/o emulsions.

8. Cosmetic and/or pharmaceutical o/w or w/o emulsions, **characterized in that** they contain 0.1 to 50% by weight of the composition claimed in any of claims 1 to 4.

## Revendications

1. Composition à base d'huile contenant :
(a) des stérols et/ou des esters de stérols, et
(b) des esters d'acides gras et de glycérol,
**caractérisée en ce qu'**
elle comporte moins de 10 % en poids d'eau, et les esters d'acides gras et de glycérol contiennent au moins 40 % de groupes acyle saturés comportant 12 atomes de carbone ou moins, et présentent un indice d'hydroxyle compris entre 20 et 120.

2. Composition selon la revendication 1,
**caractérisée en ce que**
les esters d'acides gras et de glycérol présentent une fraction de mono-glycéride d'au plus 5 %.

3. Composition selon l'une des revendications 1 à 2,
**caractérisée en ce qu'**
elle contient des stérols ou des esters de stérols d'origine végétale.

4. Composition selon au moins l'une des revendications 1 à 3,
**caractérisée en ce qu'**
elle contient de 0,01 à 10 % en poids de stérols ou d'esters de stérols.

5. Utilisation d'esters d'acides gras et de glycérol qui contiennent au moins 40 % de groupes acyle saturés comportant 12 atomes de carbone ou moins et présentant un indice OH compris entre 20 et 120, en particulier 30 et 100, pour la dissolution de stérols.

6. Utilisation de la composition selon au moins l'une des revendications 1 à 4, pour le soin cosmétique non thérapeutique de la peau.

7. Utilisation de la composition selon au moins l'une des revendications 1 à 4, pour la préparation d'émulsions H/E ou E/H cosmétiques et/ou pharmaceutiques.

8. Emulsions H/E ou E/H cosmétiques et/ou pharmaceutiques,
**caractérisées en ce qu'**
elles contiennent de 0,1 à 50 % en poids de la composition selon l'une des revendications 1 à 4.
